(19) Europäisches Patentamt
European Patent Office
Office européen des brevets

(11) Publication number: 0 276 204
A2

(12) # EUROPEAN PATENT APPLICATION

(21) Application number: 88870001.0

(22) Date of filing: 06.01.88

(51) Int. Cl.4: **A 01 N 57/16**
A 01 N 47/06, A 01 N 43/40,
C 07 D 213/80
// A01N53/00, A01N47/20,
A01N47/18, A01N47/16

(30) Priority: 07.01.87 US 1111

(43) Date of publication of application:
27.07.88 Bulletin 88/30

(84) Designated Contracting States:
AT BE CH DE ES FR GB GR IT LI LU NL SE

(71) Applicant: **Monsanto Company**
**Patent Department 800 North Lindbergh Boulevard**
**St. Louis Missouri 63167 (US)**

(72) Inventor: **Lee, Len Fang**
**2496 Annapolis Way**
**St. Charles Missouri 63303 (US)**

**Spear, Kerry Leigh**
**4530 Wichita Avenue**
**St. Louis Missouri 63110 (US)**

**Ruminski, Peter Gerrard**
**391 Crestbury**
**St. Louis Missouri 63011 (US)**

**Dhingra, Om Parkash**
**14926 Royal Brook Drive**
**Chesterfield Missouri 63017 (US)**

(74) Representative: **Lunt, John Cooper et al**
**Monsanto Europe S.A. Patent Department Avenue de Tervuren 270-272 Letter Box No 1**
**B-1150 Brussels (BE)**

(54) **Pyridine gametocides.**

(57) The present invention relates to the use of 2,6-bis(trifluoromethyl)-3-methoxycarbonyl-4-hydroxypyridine and certain derivatives thereof as gametocides.

EP 0 276 204 A2

**Description**

PYRIDINE GAMETOCIDES

FIELD OF THE INVENTION

The present invention relates to the use of certain substituted pyridines as gametocides.

BACKGROUND OF THE INVENTION

An effective gametocide is a chemical compound that, when applied to a plant during sexual development, is capable of sterilizing a plant's male gametes while leaving the plant's female gametes, or at least a significant proportion of them, capable of undergoing cross fertilization with subsequent high yields of fertile, viable hybrid seed.

The utility of a gametocide lies in the area of plant hybridization. By causing pollination of one variety of a plant species by a different variety of the same species, a hybrid plant is obtained. By careful selection of the parents, hybrids can be obtained with specific combinations of desirable traits such as plant size, grain yield, disease resistance, herbicide tolerance and climatic adaptation.

Hybridization utilizing cytoplasmic male sterility is available and is used to produce commercial hybrid corn and wheat seed. However, using such techniques, it can take years to develop lines to the point that commercial quantities of hybrid seed are produced. The use of an effective gametocide significantly reduces this development time.

Some plants, such as corn, can be easily hybridized without resort to the use of gametocides because the organ containing the male gametes are exposed and can easily be removed. This leaves the female gametes available for cross-fertilization.

However, this is not true with plants which are self-pollinating by nature such as wheat. The male and female wheat gametes are found inside the same flower which remains closed until the male anthers release their pollen onto the female gametes to fertilize them. Thus, when the flower opens, fertilization is normally complete. For a gametocide to be useful on wheat it must sterilize the male gametes and avoid interfering with the opening of the flower when the female gametes are ready for fertilization.

Substituted pyridones and pyridazines are known in the art as gametocides as disclosed in U.S. Patents 4,115,101 and 4,345,934.

However, there still is a need in the art for a more effective gametocide which effectively sterilizes the male gametes while leaving the female gametes capable of undergoing cross fertilization with a subsequent high yield of fertile viable hybrid seed.

SUMMARY OF THE INVENTION

The present invention relates to a process for selectively sterilizing the male gametes of a plant which comprises applying to a plant a compound selected from the group consisting of

(a) 2,6-bis(trifluoromethyl)-3-methoxycarbonyl-4-hydroxypyridine;

(b) agronomically acceptable salts of (a); and

(c) organic esters which are hydrolyzable to (a).

The process of the present invention can be utilized in the production of hybrid plants including monocotyledons such as wheat, barley and rice. A more thorough disclosure of the present invention is presented in the detailed description which follows.

DETAILED DESCRIPTION

The present invention relates to a process for selectively sterilizing the male gametes of a plant which comprises applying to a plant a compound selected from the group consisting of

(a) 2,6-bis(trifluoromethyl)-3-methoxycarbonyl-4-hydroxypyridine;

(b) an agronomically acceptable salt of (a); and

(c) an organic ester of (a) which is hydrolyzable to (a).

Agronomically acceptable salts useful in the process of the present invention are salts which:

(i) dissociate to 2,6-bis(trifluoromethyl)-3-methoxycarbonyl-4-hydroxy-pyridine in water, and

(ii) the salt-forming moiety that is ionically bonded to pyridine to form the salt does not have any ecologically unacceptable consequences for the plant, the soil or the general environment.

The hydroxy group of 2,6-bis(trifluoromethyl)-3-methoxycarbonyl-4-hydroxy pyridine is acidic and can form an inorganic or an organic salt when it is contacted with an inorganic or organic base. The use of a salt, particularly one in which the salt is formed by reaction with a base, is preferred since such salts are generally more water-soluble than the corresponding pyridine. This makes application of the compound significantly easier in practice. Further, it is believed that the salt of the 4-hydroxy pyridine is less volatile and therefore provides more uniform activity under varying environ mental conditions. Preferred salts are those having alkali metal cation or those having ammonium or organic ammonium cations. Other salts of the 4-hydroxy pyridine useful in the process of the present invention will be known to those skilled in the art.

A great variety of organic esters which are hydrolyzable to 2,6-bis(trifluoromethyl)-3-methoxycarbonyl-4-hydroxy pyridine or salts thereof are useful in the process of the present invention. Each of these organic esters

will have the structure of 2,6-bis(trifluoromethyl)-3-methoxycarbonyl-4-ester radical pyridine. The ester radical is hydrolyzable to the 4-hydroxy pyridine and the corresponding organic acid. For example the 4-methylcarbonyloxy ester radical is hydrolyzable to 4-hydroxy pyridine and the corresponding organic acid, acetic acid. Suitable organic esters are those in which the acid portion of the parent ester does not have any ecologically unacceptable consequences for the plant, the soil or the general environment. Suitable organic esters include oxy esters (carbonyloxy esters), thio esters, carbonates, carbamates, thiocarbamates, sulfonyl esters, sulfinyl esters, phosphate esters, phosphonate esters, phosphinate esters, phosphite and orthoesters. Suitable carbonyloxy esters have an ester radical in the 4-position of the pyridine such as alkylcarbonyloxy, cycloalkylcarbonyloxy, arylcarbonyloxy or alkoxycarbonyloxy.

Suitable oxy and thio esters have the formula:

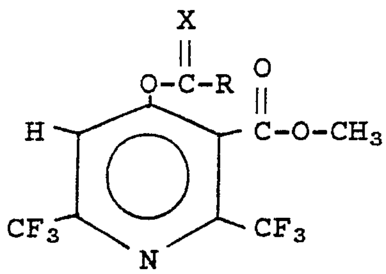

where X is S or O and R is a suitable organic radical such as hydrido, straight, branched or cyclo aliphatic hydrocarbon, aryl, alkylamino, dialkylamino, alkylphenylamino, diphenylamino, alkthio, alkoxy, phenoxy or saturated or unsaturated heterocyclic radicals contain one or more hetero atoms selected from N, O or S such as radicals as thienyl, pyrrolyl, imidazolyl, pyrazolyl, furyl, pyranyl, pyrrolyl, isothiazolyl, isoxazolyl, pyrrolidinyl, pyrrolinyl, and morpholinyl where any of such organic radical is unsubstituted or substituted with one or more of halo, hydroxy, cyano, alkoxy, nitro or the like.

Suitable sulfonyl, sulfinyl and phosphate esters have the formula:

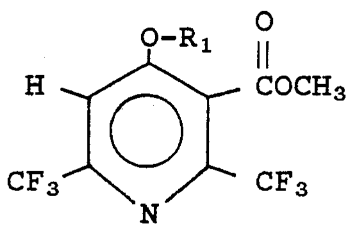

where $R_1$ is selected from the groups comprising

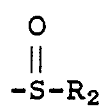

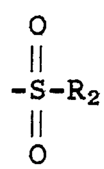

OR

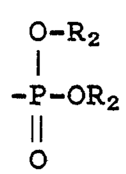

where $R_2$ is $-C(R)_3$ and each R is independently selected from the suitable organic radicals. Other organic esters suitable in the practice of the present invention will be known to those skilled in the art and may be readily adapted to the process of the present invention.

Preferred organic esters for use in the process of the present invention have the formula:

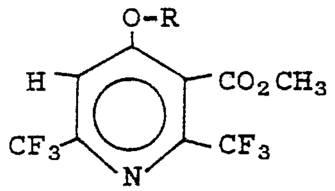

where R is tosyl, dialkoxyphosphinyl or $-\overset{X}{\overset{||}{C}}-R_1$ where X is S or O and $R_1$ is alkyl, cycloalkyl, phenyl, halo substituted phenyl, haloalkyl substituted phenyl, alkylamino, dialkylamino, alkylphenylamino, diphenylamino, pyrrolidinyl, alkoxy, alkylthio or phenoxy.

The Route A below schematically depicts a method whereby the substituted pyridine compound used in the process of this invention may be prepared from compounds which are known in the art. In this route, acetone dicarboxylic acid 1 is cyclized into the corresponding anhydride 2 by reacting it with acetic anhydride. The anhydride 2 is dissolved in methanol and stirred to form the acetone dicarboxylic acid monomethyl ester 3. The monomethyl ester is then reacted with isobutylene in acid and a suitable organic solvent to form t-butyl, methyl acetone dicarboxylate 4. The dicarboxylate 4 is then sequentially reacted with magnesium chloride in a suitable organic solvent and with trifluoroacetic anhydride to form methyl t-butyl diesterpyrone 5. The pyrone 5 is then reacted with trifluoroacetic acid to form the pyronedicarboxylic acid monoester 6. The pyrone 6 is then refluxed in a suitable organic solvent to remove the acid moiety and form the methyl ester pyrone 7. The pyrone 7 is then aminated by addition of methanol and ammonia to form 2,6-bis(trifluoromethyl)-3-(methoxycarbonyl)-4-hydroxy pyridine 8.

Route A

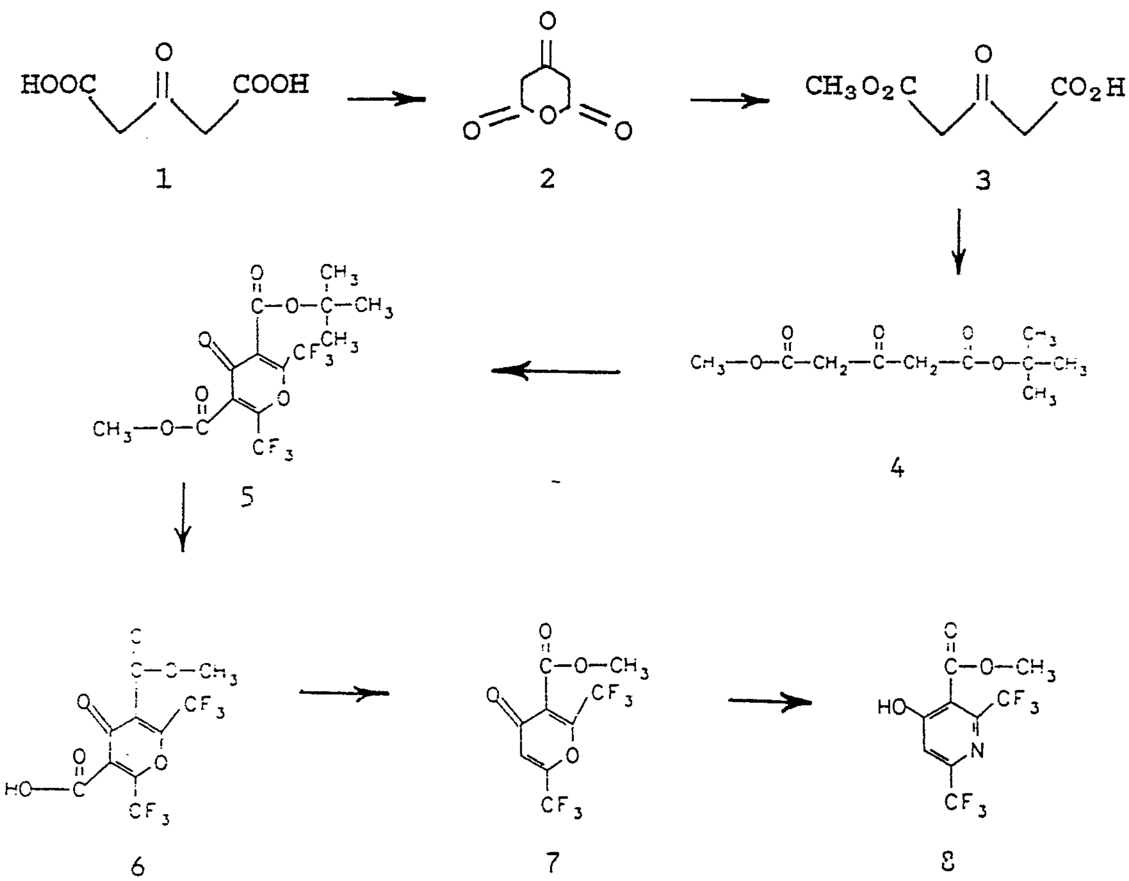

1 2 3 4 5 6 7 8

An alternative method of making the compounds used in the process of the present invention is disclosed in

EPO application 85870152.7 published November 5, 1985 and is shown in Route B below.

In Route B, a 3-ketoester (1) of the formula shown is reacted with trifluoroacetonitrile in the presence of a base. Examples of suitable bases are potassium t-butoxide, sodium in dimethoxyethane, sodium acetate, and the like. The result of this reaction is a 2-alkanoyl-3-amino-2-alkenoate ester (2); i.e., an enamine compound.

The enamine (2) compound so produced is then reacted with 2-2.5 equivalents of a strong base, suitably lithium diisopropylamide to generate in situ a dianion which is then reacted with an ester of trifluoromethyl carboxylic acid. The reaction product is a mixture of a substituted 2,6 bis(trifluoromethyl)-3-(methoxycarbonyl)-4-(hydroxy)pyridine and a substituted 2,3-dihydro-2-hydroxy-4-pyridone, which dehydrates readily when heated to form 2,6 bis(trifluoromethyl)-3-(methoxycarbonyl)-4-(hydroxy)pyridine (3).

Route B

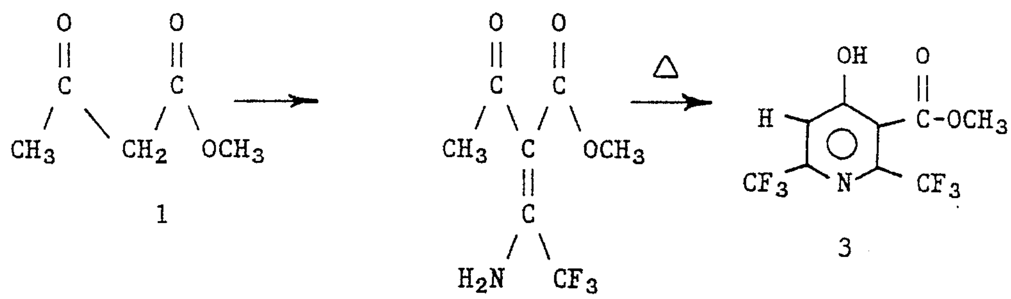

1

3

(Enamine)

2

The salts useful in the practice of the present invention may be made by reacting the 4-hydroxy pyridine with the appropriate base in a suitable organic solvent. The organic ester can generally be made by reacting the 4-hydroxy pyridine with the appropriate reactants such as acid halide, carbamyl chloride, alkyl or aryl, chloroformates or alkyl cyanate.

The following Examples 1-12 are detailed descriptions of the methods of preparing compounds useful in the practice of the present invention. These detailed descriptions fall within the scope of and serve to exemplify the more generally described method above. These Examples are presented for illustrative purposes only and are not intended as a restriction on the scope of the invention. All parts by weight unless otherwise indicated.

Example 1

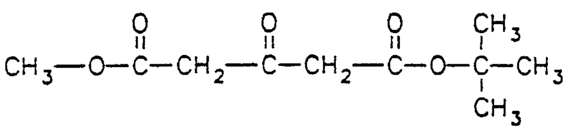

3-oxo pentanedioic acid, 1-(1,1-dimethylethyl)-5-methyl ester

To acetic anhydride (350 ml) at 0° acetone dicarboxylic acid (200 g, 1.37 mole) was added slowly over 1/2 hour with vigorous stirring. After stirring 3 hours, a white solid was filtered and washed with ether (100 ml). Drying the solid 3 hours at 50° C (0.1 mm) gave acetone dicarboxylic acid anhydride (149 g), m.p. 134-136° C.

The acetone dicarboxylic acid anhydride (20 g) just prepared was dissolved in methanol (80 ml) causing an exotherm. After stirring 1 1/2 hours, contents were concentrated in vacuo and the residue was dried under high vacuum to give a light brown oil: acetone dicarboxylic acid, monomethyl ester (24.0 g).

The acetone dicarboxylic acid, monomethyl ester (24.0 g) just prepared was mixed with ether (20 ml), conc $H_2SO_4$ (1 ml) and condensed isobutylene (35 g) in a 500 ml pressure bottle. Contents were stoppered and shaken mechanically on a Parr hydrogenation apparatus for 20 hours. After releasing the excess isobutylene, the contents were diluted with ether (150 ml) and washed with water (2 × 25 ml), sat'd aqueous $NaHCO_3$ (4 × 25 ml), water (2 × 25 ml) and brine (1 × 50 ml). The ether layer was dried over $Na_2SO_4$ and concentrated in vacuo leaving an amber oil. Distillation at 103-105° C (2.6 mm) gave a colorless oil, 21.1 g (63%).

Elemental analysis for $C_{10}H_{16}O_5$

| | C | H |
|---|---|---|
| Calculated | 55.55 | 7.46 |
| Found | 55.58 | 7.46 |

Example 2

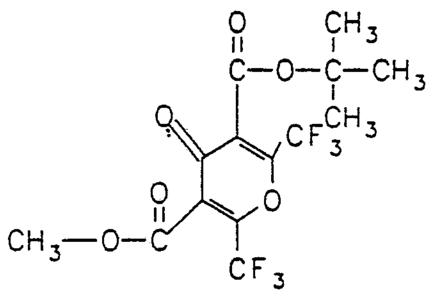

2H-pyran-3,5-dicarboxylic acid, 2,6-bis(trifluoromethyl)-3-methyl-5-(1,1-dimethylethyl)ester

The pentanedioic acid diester from Example 1 (10.8 g. 0.05 mole), anhydrous magnesium chloride (4.8 g 0.05 mole) and pyridine (8 ml) were mixed in anhydrous THF (15 ml) and refluxed for one hour. After cooling contents to -5°C, trifluoroacetic anhydride (28.3 ml, 0.2 mole) was added dropwise over 15 minutes, keeping the temperature less than 0°. Stirred for one hour at 0-5° and then contents were concentrated in vacuo. The residue (oily solid) was taken up in $CH_2Cl_2$ and washed with water (2 × 500 ml), dried over $MgSO_4$ and concentrated in vacuo, to give a light yellow solid (16.8 g). The solid was twice recrystallized from cyclohexane to give a white solid, 8.8 g (49%): m.p. 114-117°C.

Elemental analysis for $C_{14}H_{12}F_6O_6$

| | C | H |
|---|---|---|
| Calculated | 43.09 | 3.10 |
| Found | 43.09 | 3.05 |

Example 3

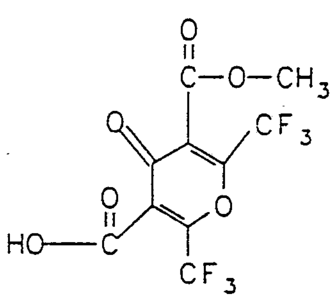

2H-pyran-3,5-dicarboxylic acid, 4-oxo-2,6-bis(trifluoromethyl)-3-methyl ester

The ester from Example 2 (21.1 g, 0.054 mole) and trifluoroacetic acid (25 ml) were stirred magnetically for 2 hours. Contents were concentrated in vacuo and the residue left under high vacuum to remove the last traces of trifluoroacetic acid. $CH_2Cl_2$ was added to the residue and a solid was filtered. Recrystallization from toluene gave a white solid 14.0 g (78%): m.p. 160-163°C.

Elemental analysis for $C_{10}H_4F_6O_6$

| | C | H |
|---|---|---|
| Calculated | 35.47 | 1.34 |
| Found | 35.50 | 1.30 |

Example 4

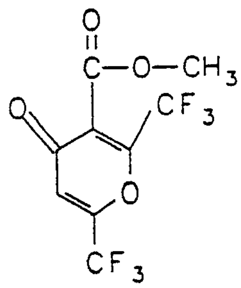

4H-pyran-3-carboxylic acid, 4-oxo-2,6-bis(trifluoromethyl), methyl ester

The ester from Example 3 (5 g, 0.015 mole) was refluxed in xylene (100 ml) for 3 hours. After cooling, contents were concentrated in vacuo and the residue, a light amber oil, was purified by kugelrohr distillation at 50° (0.15 mm) to give a white solid, 3.5 g (80%): m.p. 36-38°C.

Elemental analysis for $C_9H_4F_6O_4$

| | C | H |
|---|---|---|
| Calculated | 37.26 | 1.39 |
| Found | 37.25 | 1.48 |

Example 5

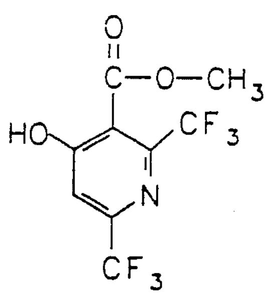

2,6-bis(trifluoromethyl)-3-methoxycarbonyl-4-hydroxy pyridine

To the ester of Example 4 (28.5 g, 0.1 mole) was added a solution of methanol containing anhydrous ammonia (approximately 100 ml), causing an exotherm and immediate dissolution. After stirring for 0.5 hours, contents were concentrated in vacuo. The residue was dissolved in water (200 ml) and extracted with ether. The water layer was made acidic to pH 7.0 with 2N HCl, and again extracted with ether. The water layer was made more acidic to pH 2.0 and extracted with ether. This last ether extraction was dried over $MgSO_4$ and concentrated in vacuo leaving a solid (25.3 g). The solid was recrystallized from hexane to yield 24.0 g (83%) of the product as a white solid m.p. 62-75°C.

Elemental analysis for $C_9H_5F_6NO_3$

| | C | H |
|---|---|---|
| Calculated | 37.38 | 1.74 |
| Found | 37.35 | 1.74 |

Example 6

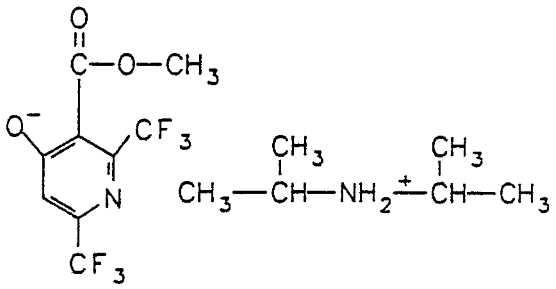

Diisopropylamine salt of 2,6-bis(trifluoromethyl)-3-(methoxycarbonyl)-4-hydroxy pyridine.

To a stirred solution of 1.5 g (5 mmole) of the ester in Example 5 in 15 ml of ether was added dropwise 0.5 g (5 mmole) of diisopropylamine. After stirring 10 minutes, the contents were concentrated in vacuo leaving a white solid. The solid was recrystallized from ether-hexane to yield 1.6 g (82%) of the product as a white solid. M.P. 94-104°C.

Elemental analysis for $C_{15}H_{20}F_6N_2O_3$

| | C | H |
|---|---|---|
| Calculated | 46.16 | 5.16 |
| Found | 46.22 | 5.25 |

Example 7

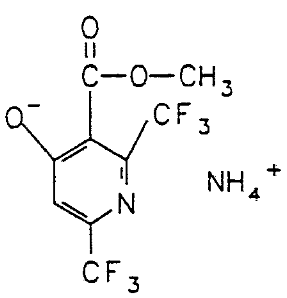

The ammonium salt of 2,6-bis(trifluoromethyl)-3-methoxycarbonyl-4-hydroxy pyridine

45 g (0.135 mole) of the ester of Example 3 in 100 ml of xylene was refluxed for 4 hrs. After cooling to room temperature and then stirring overnight, 400 ml of $CH_3OH$ was added to the reaction mixture. 5 to 6 g (2.5-3 equiv.) of $NH_3$ was then bubbled into the above solution. This was then stirred overnight at room temperature. The solvents were removed under vacuum and $CH_2Cl_2$ was added and the mixture stirred for 1 hr. The ppt. was filtered and dried to yield 38.5 g (93%) of product as a light yellow solid. M.P. 187-189°C.

Elemental analysis for $C_9H_8F_6N_2O_3$

| | C | H | N |
|---|---|---|---|
| Calculated | 35.31 | 2.63 | 9.15 |
| Found | 35.36 | 2.62 | 9.12 |

Example 8

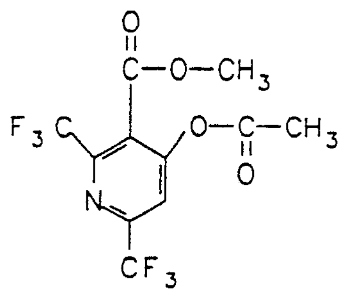

2,6-bis(trifluoromethyl)-3-(methoxycarbonyl)-4-(acetyloxy) pyridine

To 4 g (0.0138 mole) of the pyridine of Example 5 dissolved in 60 ml of ether is added 1.4 g (0.0138 mole) of triethylamine. 1.09 g (0.0138 mole) of acetyl chloride in 10 ml of ether is then added dropwise to the reaction mixture at 0° C (white ppt. forms). This reaction mixture is stirred overnight at room temperature. The salts are filtered off and the ether is removed from the filtrate under vacuum. The resulting residue solidified and this was recrystalized from cyclohexane to yield 3.6 g (80%) of a white solid. M.P. = 63-4° C.

Elemental analysis for $C_{11}H_7F_6NO_4$

| | C | H | N |
|---|---|---|---|
| Calculated | 39.89 | 2.13 | 4.23 |
| Found | 39.89 | 2.14 | 4.19 |

Example 9

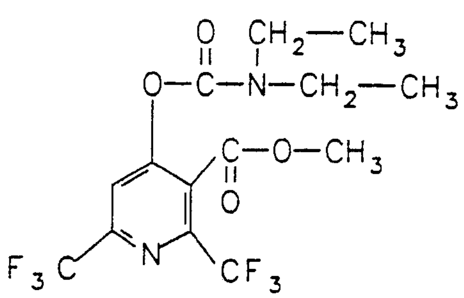

2,6-bis(trifluoromethyl)3-(methoxycarbonyl)-4-(diethylcarbamyloxy) pyridine

To 2 g (0.0069 mole) of the pyridine of Example 5 in 20 ml of $CH_3CN$ is added 0.7 g (0.0069 mole) of triethylamine. 0.94 g (0.069 mole) of diethylcarbamyl chloride is added and stirred at room temperature over the weekend and then reflux for 5 hrs. The solvent is removed under vacuum and the crude solid is recrystallized from cyclohexane (taking nearly a week for total crystallization to occur). The recrystallized

product is dried on a porous plate and then under vacuum to yield 1.2 g (45%) of a white solid. M.P. = 47-49°C.

Elemental analysis for $C_{14}H_{14}F_6N_2O_4$

| | C | H | N |
|---|---|---|---|
| Calculated | 43.31 | 3.63 | 7.21 |
| Found | 43.41 | 3.64 | 7.19 |

Example 10

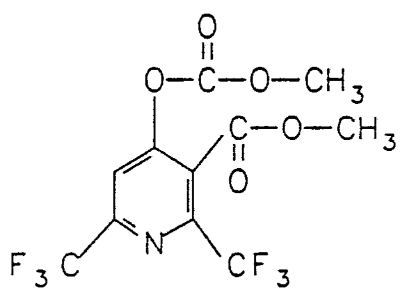

2,6-bis(trifluoromethyl)-3-(methoxycarbonyl)-4-(methoxycarbonyloxy) pyridine

Methyl chloroformate (1.63 g, 0.0173 mole) and ether (10 ml) were added dropwise at 0°C to a solution of the pyridine of Example 5 (5 g, 0.0173 mole) and triethylamine (2.41 ml) in 50 ml ether. After 24 hours of stirring, the precipitate was filtered off and the filtrate was concentrated in vacuo leaving a solid which was recrystallized in cyclohexane and dried in vacuo to give 4.57 g of white crystals (76%): M.P. = 61-63°C, moisture sensitive.

Elemental analysis for $C_{11}H_7F_6NO_5$

| | C | H | N |
|---|---|---|---|
| Calculated | 38.06 | 2.03 | 4.03 |
| Found | 38.04 | 2.04 | 4.00 |

Example 11

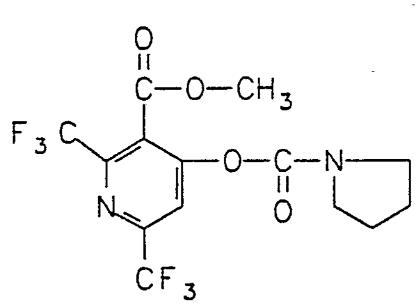

2,6-bis(trifluoromethyl)-3-(methoxycarbonyl)-4-(1-pyrrolidinecarbonyloxy) pyridine

To 2 g (0.0069 mole) of the pyridine of Example 5 dissolved in 20 ml of $CH_3CN$ is added 0.7 g (0.0069 mole) of triethylamine. 0.92 g (0.0069 mole) of 1-pyrrolidinecarbonyl chloride was then added to the reaction mixture

and this was then stirred overnight at room temperature then at reflux for 6 hrs. Solvents were then removed under vacuum and the residue slurried in ether. The resulting salts were filtered off and the ether removed from the filtrate under vacuum. The residue solidified after being put under vacuum overnight and this crude product was recrystallized from cyclohexane, dried on a porous plate and then dried under vacuum to yield 1.80 g (68%) of a white solid. M.P. = 73-75°C.

Elemental analysis for $C_{14}H_{12}F_6N_2O_4$

| | C | H | N |
|---|---|---|---|
| Calculated | 43.53 | 3.13 | 7.25 |
| Found | 43.61 | 3.18 | 7.22 |

Example 12

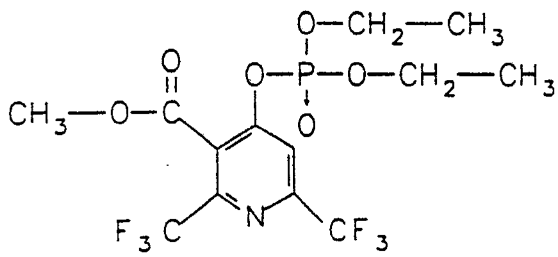

4-{(diethoxyphosphinyl)oxy}-2,6-bis(trifluoromethyl)-3-pyridine carboxylic acid, methyl ester

To 4 g (0.014 mole) of the ester of Example 5 in 60 ml of ether was added 1.4 g (0.014 mole) of triethyl amine and contents were cooled to 0°C. A solution of 2.4 g (0.014 mole) of diethyl chlorophosphate in 10 ml of ether was added dropwise at 0°C causing a white precipitate. After stirring at room temperature for 3.5 hours, the precipitate was filtered. The ether filtrate was washed with 1N HCl, 3 × 0.5N NaOH, 2 × brine, dried over $MgSO_4$ and concentrated in vacuo to yield 5.3 g (90%) of product as a light yellow oil. B.P. 120°C (0.2 mm).

Elemental analysis for $C_{13}H_{14}N_1O_6P_1F_6$

| | C | H |
|---|---|---|
| Calculated | 36.72 | 3.32 |
| Found | 36.86 | 3.35 |

Using the appropriate starting materials and generally following the procedures of Examples 6 through 12, the following compounds were prepared.

Table I

| Example Compound # | Name | Structure | Analysis (%) Calc'd | Analysis (%) Found | M.P./B.P. °C |
|---|---|---|---|---|---|
| 13 | isopropylamine salt of 2,6-bis(trifluoromethyl)-3-(methoxycarbonyl)-4-(hydroxy)pyridine | 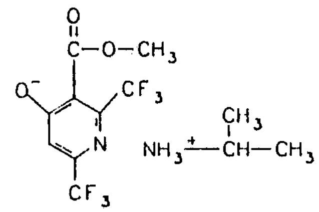 | C 41.39<br>H 4.05 | 41.45<br>4.08 | 152-154 |
| 14 | cyclohexylamine salt of 2,6-bis(trifluoromethyl)-3-(methoxycarbonyl)-4-(hydroxy)pyridine | 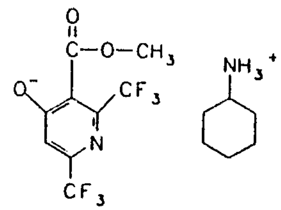 | C 46.40<br>H 4.67 | 46.41<br>4.71 | 135-137 |
| 15 | t-butylamine salt of 2,6-bis(trifluoromethyl)-3-(methoxycarbonyl)-4-(hydroxy)pyridine | 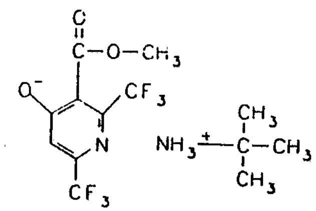 | C 43.10<br>H 4.45 | 43.13<br>4.48 | 141-143 |
| 16 | dicyclohexylamine salt of 2,6-bis(trifluoromethyl)-3-(methoxycarbonyl)-4-(hydroxy)pyridine | 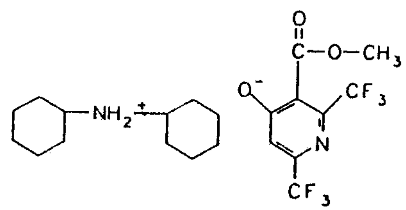 | H 53.61<br>H 6.00 | 53.71<br>5.91 | 183-185 |

Table I (Con't.)

| Example Compound # | Name | Structure | Analysis (%) Calc'd | Found | M.P./B.P. °C |
|---|---|---|---|---|---|
| 17 | sodium salt of 2,6-bis(trifluoromethyl)-3-(methoxycarbonyl)-4-(hydroxy)pyridine | 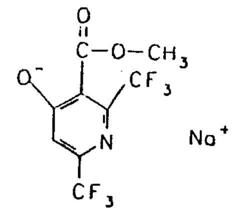 | C 34.74<br>H 1.30 | 34.68<br>1.34 | 272-273 |
| 18 | 2,6-bis(trifluoromethyl)-3-(methoxycarbonyl)-4-(t-butylcarbonyloxy)pyridine | 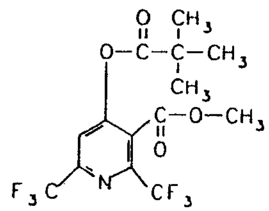 | C 45.05<br>H 3.51 | 44.91<br>3.52 | |
| 19 | 2,6-bis(trifluoromethyl)-3-(methoxycarbonyl)-4-(propylcarbonyloxy)pyridine | 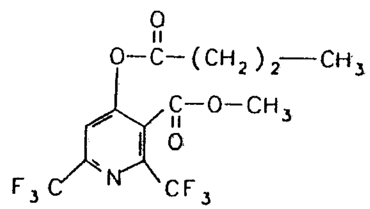 | C 43.47<br>H 3.09 | 43.40<br>3.13 | |
| 20 | 2,6-bis(trifluoromethyl)-3-(methoxycarbonyl)-4-(isopropylcarbonyloxy)pyridine | 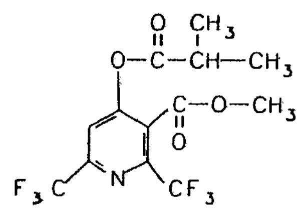 | C 43.47<br>H 3.09 | 43.45<br>3.11 | |

## Table I (Con't.)

| Example Compound # | Name | Structure | Analysis (%) Calc'd | Found | M.P./B.P. °C |
|---|---|---|---|---|---|
| 21 | 2,6-bis(trifluoromethyl)-3-(methoxycarbonyl)-4-(cyclohexylcarbonyloxy)pyridine | 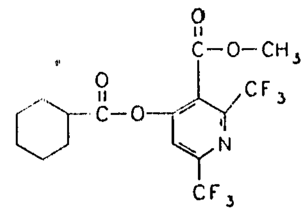 | C 48.13<br>H 3.79 | 48.24<br>3.79 | |
| 22 | 2,6-bis(trifluoromethyl)-3-(methoxycarbonyl)-4-(cyclopropylcarbonyloxy)pyridine | 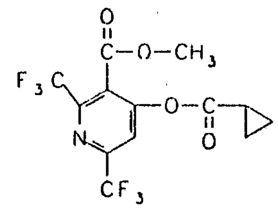 | C 43.71<br>H 2.54 | 43.77<br>2.55 | 71-72 |
| 23 | 2,6-bis(trifluoromethyl)-3-(methoxycarbonyl)-4-(benzoxy)pyridine | 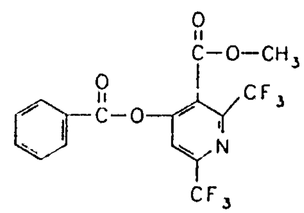 | C 48.87<br>H 2.31 | 48.95<br>2.34 | 121-122 |
| 24 | 2,6-bis(trifluoromethyl)-3-(methoxycarbonyl)-4-(pentafluorobenzoxy)pyridine | 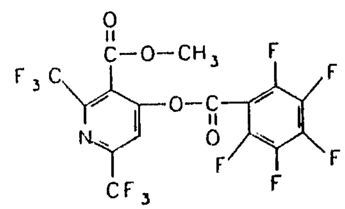 | C 39.77<br>H 0.83 | 39.71<br>0.97 | 55-57 |

Table I (Con't.)

| Example Compound # | Name | Structure | Analysis (%) | Calc'd | Found | M.P./B.P. °C |
|---|---|---|---|---|---|---|
| 25 | 2,6-bis(trifluoro-methyl)-3-(methoxycarbonyl)-4-(4'-trifluoromethylbenzoyloxy)-pyridine | 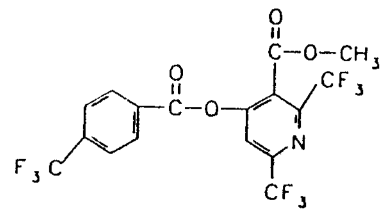 | C<br>H | 44.27<br>1.75 | 44.12<br>1.74 | 79-81 |
| 26 | 2,6-bis(trifluoro-methyl)-3-(methoxycarbonyl)-4-(dimethylcarbamyloxy)pyridine | 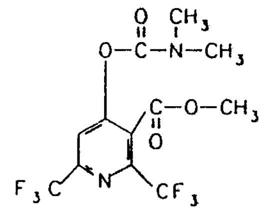 | C<br>H | 40.01<br>2.80 | 40.07<br>2.81 | 58-59 |
| 27 | 2,6-bis(trifluoro-methyl)-3-(methoxycarbonyl)-4-(dipropylcarbamyloxy)pyridine | 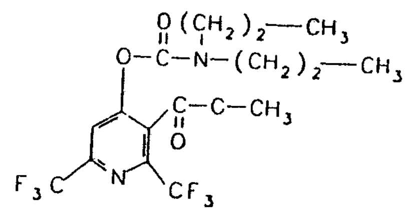 | C<br>H | 46.16<br>4.36 | 46.21<br>4.38 | 62-64 |
| 28 | 2,6-bis(trifluoro-methyl)-3-(methoxycarbonyl)-4-(phenylmethylcarbamyloxy)pyridine | 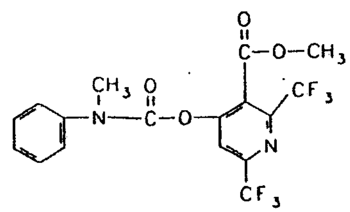 | C<br>H | 48.35<br>2.86 | 48.42<br>2.97 | 60-63 |

Table I (Con't.)

| Example Compound # | Name | Structure | | Analysis (%) Calc'd | Found | M.P./B.P. °C |
|---|---|---|---|---|---|---|
| 29 | 2,6-bis(trifluoromethyl)-3-(methoxycarbonyl)-4-(diisopropylcarbamyloxy)pyridine | 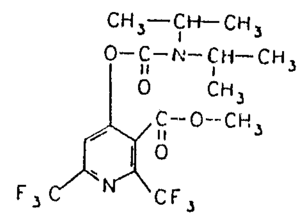 | C<br>H | 46.16<br>4.36 | 46.09<br>4.40 | 59-61 |
| 30 | 2,6-bis(trifluoromethyl)-3-(methoxycarbonyl)-4-(diphenylcarbamyloxy)pyridine | 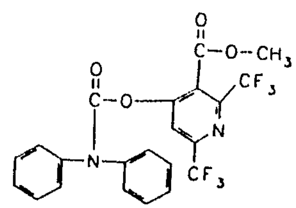 | C<br>H | 54.55<br>2.91 | 54.69<br>3.02 | 114-116 |
| 31 | 2,6-bis(trifluoromethyl)-3-(methoxycarbonyl)-4-(phenoxycarbonyloxy)pyridine | 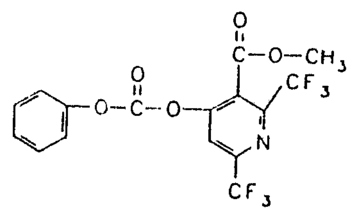 | C<br>H | 46.96<br>2.22 | 47.00<br>2.26 | 93-94 |
| 32 | 2,6-bis(trifluoromethyl)-3-(methoxycarbonyl)-4-(ethylthiocarbonyloxy)pyridine | 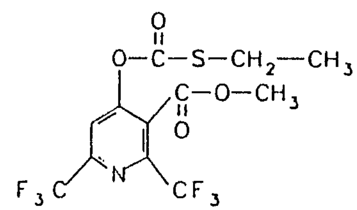 | C<br>H | 38.20<br>2.40 | 38.23<br>2.41 | 50-52 |

Table I (Con't.)

| Example Compound # | Name | Structure | Analysis (%) Calc'd | Found | M.P./B.P. °C |
|---|---|---|---|---|---|
| 33 | 2,6-bis(trifluoro-methyl)-3-(methoxycarbonyl)-4-(dimethylaminocarbonothioyloxy)-pyridine | 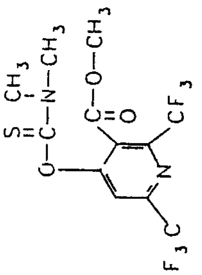 | C 38.30<br>H 2.68 | 38.33<br>2.69 | 76-79 |
| 34 | 2,6-bis(trifluoro-methyl)-3-(methoxycarbonyl)-4-(4'-methylphenylsulfonyloxy)pyridine | 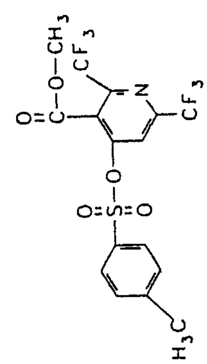 | C 43.35<br>H 2.50 | 43.48<br>2.64 | 38-40 |

In the process of the present invention a sterilizing effective amount of the gametocide should preferably be applied after initiation of sexual development (e.g. spike formation), but prior to sexual maturity, that is before

pollen is shed from the anthers. Generally, application in wheat should occur when the spike or head length is from about 1 to 9 cm, preferably about 3 to 7 cm and most preferably about 5 cm. In other plants application should preferably be when the plants have reached a corresponding degree of sexual maturity. It has been found that application of the compound early in the day generally results in both greater gametocide activity and plant injury. It is generally preferred to apply the compound to the plant later in the day.

In the process of the present invention the gametocide sterilizes most male gametes while leaving a significant portion of the female gametes capable of fertilization. Further, the gametocide has a minimum effect on the rest of the plant (e.g. stunting, chlorosis etc.).

In the process of the present invention, the gametocide is applied at the appropriate time at a rate of about 1 to about 20 lbs/a preferably about 7 to about 12 lbs/a. It will be obvious to those skilled in the art that the optimum application rate for the gametocide will depend on a variety of factors such as plant type, soil type, fertility, environmental factors and the like and those skilled in the art will be capable of readily determining the optimum rate based on the Examples herein and their experience. Spray volumes may be varied substantially e.g., from 30 gal/a to 300 gal/a without any appreciable effect on activity of the compounds. It is believed that the effectiveness of the organic esters used in the process of the present invention is in part a function of the ease in which they undergo hydrolysis to the 4-hydroxy pyridine.

In the process of the present invention, the compound is applied at the appropriate time to the plant locus. Application to the plant locus include application to the foliage and to the plant growth medium e.g., soil to enable assimilation of the chemical into the growing plant. The compounds in the process of the present invention can be used with adjuvants in liquid or solid form. The compositions are prepared by admixing the compounds with an adjuvant including diluents, extenders, carriers, and conditioning agents to provide compositions in the form of finely-divided particulate solids, granules, pellets, solutions, dispersions, or emulsions. Thus, they can be used with an adjuvant, such as a finely-divided solid, a liquid of organic origin, water, a wetting agent, binder, carrier, or any suitable combination of these.

The compounds can also be used in the form of liquids and wettable powders. These preferably contain as a conditioning agent one or more surface-active agents in amounts sufficient to render a given compound readily dispersible in water or in oil. The incorporation of a surface-active agent into the compound can enhance its efficacy. By the term "surface-active agent" it is understood that wetting agents, dispersing agents, suspending agents, and emulsifying agents are included therein. Anionic, cationic, and nonionic agents can be used.

It is believed that suitable wetting agents are alkyl benzene and alkyl naphthalene sulfonates, sulfonated fatty alcohols, amines or acid amides, long chain acid esters of sodium isothionate, esters of sodium sulfosuccinate, sulfated or sulfonated fatty acid esters, petroleum sulfonates, sulfonated vegetable oils, ditertiary acetylenic glycols, polyoxyethylene derivatives of alkylphenyls (particularly isooctylphenol and nonylphenol) and polyoxyethylene derivatives of the mono-higher fatty acid esters of hexitol anhydrides (e.g., sorbitan). Preferred surfactants are dihexyl ester of sodium sulfosuccinic acid, POE 20 sorbitan monolaurate and octylphenoxy polyethoxy ethanol.

Wettable powders or dispersable granules are water-dispersible compositions containing one or more active ingredients, an inert solid extender, and one or more wetting and dispersing agents. The inert solid extenders are usually of mineral origin, such as the natural clays, diatomaceous earth, salts and synthetic minerals, derived from silica and the like. Examples of such extenders include kaolinites, attapulgite clay, salts and synthetic magnesium silicate.

Aqueous suspensions or emulsions may be prepared by stirring an aqueous mixture of a water-insoluble active ingredient and an emulsification agent until uniform and then homogenized or milled to give stable emulsion or suspension of very finely-divided particles. The resulting concentrated aqueous suspension is characterized by its extremely small particle size, so that when diluted and sprayed, coverage is very uniform.

Concentrates are usually solutions of the active ingredients in water-immiscible or partially water-immiscible solvents together with a surface active agent. Suitable solvents for the active ingredients of this invention will be known to those skilled in the art.

Granules are physically stable particulate compositions comprising the active ingredients adhering to or distributed through a basic matrix of an inert, particulate extender. In order to aid leaching of the active ingredient from the particulate, a surface active agent, such as those listed hereinbefore, can be present in the composition. Natural clays, pyrophyllites, illite, gypsum, and vermiculite are examples of operable classes of particulate mineral extenders. The preferred extenders are the porous absorptive, preformed particles, such as preformed and screened particulate attapulgite or heat-expanded, particulate vermiculite, and the finely-divided clays, such as kaolin clays, hydrated attapulgite, or bentonitic clays. These extenders are sprayed or blended with the active ingredient to form the herbicidal granules.

In order to avoid the time consuming effort of field testing a very large number of chemical compounds as potential gametocides, the art skilled have developed various bioactivity screens to identify chemical compounds which may exhibit gametocide activity.

A primary screen for wheat involves applying a chemical compound to a plant at the appropriate time when the spike length of the primary tillers is about 1 to 9 cm preferably from about 3 to 7 cm and most preferably about 5 cm, and observing the effect on the plant. Potential gametocide activity is evidenced by the flower opening wide which normally only occurs after the pollen from the male gamete has been discharged onto the female gamete. This is known as showing "open head morphology". Further, the compound should show only

a slight stunting of the plant.

Chemical compounds which show suitable open head morphology in the primary screen are then tested in a secondary screen. The secondary screen generally involves applying the chemical compound to the wheat plant when the spike has reached the appropriate length and then bagging a portion of the plant heads. When seeds are large enough to be seen, the spikes are evaluates for the number of seed set. The following examples are illustrative.

Example 35

The process of the present invention is illustrated by testing the gametocides of Examples 5-34 utilizing the following general procedure. Awned hard red spring wheat cultivar Anza was grown in a growth chamber set at low humidity (70%), at 19° C:17° C (day:night) temperatures with a 16 hour photoperiod, and at a light intensity of 800uE. Seven seeds per six inch pot were planted in a soilless medium of Metro-Mix200 supplemented with 93 g Osmocote (14-14-14) Controlled Release Fertilizer, 93 g Peters (14-7-7) Slow Release Fertilizer, 17 g Micromax Micronutrients per cubic foot of Metro-Mix200. Approximately 10-14 days after planting, pots were thinned to six plants per pot. Chemical treatments were foliar applied using an enclosed track sprayer chamber. This consists of a compressed air reservoir equipped with a manual speed adjustment and a field sprayer nozzle. This method simulated actual field spraying techniques. The test consisted of using three rates on one date in a carrier solution of 50% Acetone-50% $H_2O$-0.2%Tween20 sprayed at a rate equivalent to 300 gal/acre. On the spray date the mean spike length of primary tillers averaged about 4 to $5^1/_2$ cm. Controls treated with carrier solution were included for comparison. After spike emergence and before anthesis, glassine bags were placed over approximately 12-15 heads per pot to prohibit all but self-pollination. When seeds were large enough to be easily seen (approximately 4 weeks after heading), bagged spikes were evaluated for the number of seed set. In most cases the data is the average of two or more replicas. In all of the tests the controls were all fertile.

| Example No. | Spray Rates 1 | 2 | 3 |
|---|---|---|---|
| | (means % of sterility) | | |
| 5 | 65 | 91 | 61* |
| | 71 | 100 | 100*** |
| 6 | 79 | 99 | 99* |
| | 85 | 99 | 94* |
| 7 | 67 | 90 | 90*** |
| 8 | 61 | 100 | 83*** |
| 9 | 55 | 69 | 70** |
| 10 | 59 | 70 | 58* |
| 11 | 64 | 84 | 82** |
| 12 | 53 | 94 | 96* |
| 13 | 99 | 99 | 100** |

| Spray Rates (lb/A) 1 | 2 | 3 |
|---|---|---|
| *3.1 | 9.3 | 15.5 |
| ** 4 | 8 | 12 |
| *** 5 | 10 | 15 |

| Example No. | Spray Rates 1 | 2 | 3 |
|---|---|---|---|
| | (means % of sterility) | | |
| 14 | 100 | 100 | 100** |
| 15 | 99 | 99 | 100** |
| 16 | 100 | 96 | 100** |
| 17 | 87 | 99 | 99** |
| 18 | 19 | 62 | 85*** |
| 19 | 61 | 100 | 92*** |
| 20 | - | 93 | 97*** |
| 21 | 64 | 92 | 100*** |
| | 80 | 94 | 97** |
| 22 | 52 | 87 | 92*** |
| 23 | 30 | 53 | 46*** |
| 24 | 60 | 81 | 91* |
| 25 | 68 | 97 | 94* |
| 26 | 50 | 74 | 74** |
| 27 | 27 | 0 | 52** |
| 28 | 82 | 86 | 84** |
| 29 | 35 | 65 | 75** |
| 30 | 30 | 15 | 24** |
| 31 | 91 | 89 | 96* |
| 32 | 53 | 74 | 65* |
| 33 | 48 | 57 | 75** |
| 34 | 76 | 87 | 99* |

| Spray Rates (lb/A) 1 | 2 | 3 |
|---|---|---|
| *3.1 | 9.3 | 15.5 |
| ** 4 | 8 | 12 |
| *** 5 | 10 | 15 |

Example 36

The compound of Example 6 was tested on awned spring barley cultivar Steptoe generally in accordance with the procedure of Example 35 with a 12 hour photoperiod with the following results.

| Spray Date | Spike Length (cm) | Spray Rates (lb/A) 5.0 | 7.5 | 10.0 | 12.5 |
|---|---|---|---|---|---|
| | | (means % of sterility) | | | |
| D1 | 1.65 | 54* | 61 | 58 | 40* |
| D2 | 3.02 | 78 | 84 | 86 | 82 |
| D3 | 4.82 | 67 | 75 | 71 | 81 |
| D4 | 6.14 | 67 | 70 | 86 | 81 |

* only one of the two replications show sterility.

In order to further show the present invention, wheat plants were treated in accordance with the process of the present invention; cross-fertilized and the hybrid seed tested for seed germination generally in accordance with the following procedure.

The compounds were tested on field grown winter wheat plants. Experimental plots were four feet long and seven rows wide consisting of both male and female rows. The female rows (spray rows) were the center three rows and consisted of one awned (B393) and one awnless (Caldwell) winter wheat cultivars containing either the Rht1 or Rht2 dwarfing gene. The female rows were bordered by two male rows (the pollen source). The male rows containing a mixture of cultivars, which varied in date of anthesis and awn type. The cultivars did not contain either of the dwarfing genes. All of the cultivars were seeded with a Marliss grain drill at an approximate seeding rate of 1.5 million seeds per acre (approximately 1.3 bushels per acre which is standard for the area and growing conditions. Fertilizers, insecticides and fungicides were applied generally in accordance with good farming practice.

Timing of the gametocide application was based on spike length and ranged from 1.0 cm to 7.0 cm which would make the stage of plant development from the jointing to the late boot stage. Spikes were excised from the most mature tillers at regular intervals with the spike lengths being used to monitor spike development.

Treatments were foliar-applied to the female rows using a spray boom mounted to a high-clearance tractor with a single spray nozzle directly above each of the spray rows. Treatment chemicals were formulated in water or acetone and water. All formulations included 0.2% Tween20 as a surfactant. Applications were made with a solvent volume equivalent to approximately 80 gallons per acre.

Glassine bags were placed on 20-25 spikes per plot to determine the level of male sterility. The glassine bags were placed on the spikes after head emergence, but prior to anthesis (anther extrusion). Approximately four weeks later after the seeds were in a soft-hard dough stage of development, the bagged spikes were collected and scored for seed set.

Grain yield was determined following harvesting the entire female plot with a Yanmar crop binder and threshing with a Vogel thresher. A sub-sample of the grain was used in a standard gibberellin (GA3) test to determine hybrid seed set. The GA3 test consisted of planting 75 seeds from each plot into plastic pans containing a soil-less medium of Metromix-200. Each pan contained either a male (gibberellic acid-GA3 responsive) or a female (GA3 non-responsive) control. Plants were scored for responsiveness to GA3 approximately two weeks (15-16 days) after seeding by comparing the plant height at the second leaf node to either the responsive (male) or the non-responsive (female) controls. Hybrid seed produced by cross pollination with pollen from the male rows onto the sterile female stigmas would develop seedlings with elongated internodes when grown in the GA3 solution.

The spray date of the gametocide was correlated with spike length as follows:

| Spray Date | Spike Length (cm) |
|---|---|
| Cultivar B393 | |
| D1 | 1.7 |
| D3 | 3.3 |
| D5 | 4.5 |
| D7 | 8.0 |
| Cultivar Caldwell | |
| D1 | 1.4 |
| D3 | 3.7 |
| D5 | 4.2 |
| D7 | 8.0 |

Example 37

The compound of Example 5 was tested in accordance with the foregoing described procedure with the following results:

| Treatment Date | Treatment Rate (lb/A) | Caldwell Sterility (%) | Caldwell Grain Yield (bu/A) | Caldwell Hybrid Seed (%) | Caldwell Seed Germination (%) | B393 Sterility (%) | B393 Grain Yield (bu/A) | B393 Hybrid Seed (%) | B393 Seed Germination (%) |
|---|---|---|---|---|---|---|---|---|---|
| 1) Control | | 0 | 78.5 | 0.0 | 81.3 | 0 | 75.3 | 0.1 | 88.0 |
| 2) D1 | 2.50 | 28 | 51.1 | 7.0 | 78.7 | 44 | 62.3 | 9.0 | 86.2 |
| D1 | 5.00 | 51 | 44.5 | 9.3 | 82.7 | 62 | 50.8 | 7.0 | 86.7 |
| D1 | 7.50 | 30 | 53.9 | 4.0 | 77.8 | 50 | 46.5 | 5.0 | 87.6 |
| D1 | 10.00 | 46 | 41.3 | 5.0 | 81.3 | 64 | 38.7 | 14.0 | 80.4 |
| D1 | 12.50 | 49 | 42.2 | 11.3 | 83.1 | 60 | 41.1 | 15.7 | 84.4 |
| 3) D3 | 2.50 | 40 | 55.3 | 7.3 | 83.1 | 68 | 61.5 | 10.7 | 88.4 |
| D3 | 5.00 | 53 | 41.2 | 11.0 | 70.7 | 71 | 52.2 | 17.0 | 91.6 |
| D3 | 7.50 | 83 | 29.3 | 20.0 | 79.6 | 53 | 45.2 | 14.0 | 87.1 |
| D3 | 10.00 | 97 | 21.6 | 32.7 | 70.2 | 66 | 45.4 | 18.7 | 90.2 |
| D3 | 12.50 | 72 | 28.4 | 12.0 | 84.4 | 71 | 42.2 | 16.3 | 80.0 |
| 4) D5 | 2.50 | 20 | 61.7 | 3.0 | 77.8 | 49 | 58.4 | 16.3 | 87.1 |
| D5 | 5.00 | 45 | 43.3 | 8.7 | 76.9 | 84 | 50.3 | 26.0 | 85.8 |
| D5 | 7.50 | 55 | 34.4 | 11.0 | 70.7 | 83 | 41.1 | 32.3 | 81.8 |
| D5 | 10.00 | 48 | 36.9 | 10.0 | 76.4 | 88 | 40.2 | 29.3 | 79.6 |
| D5 | 12.50 | 49 | 25.0 | 24.7 | 53.3 | 88 | 31.6 | 42.0 | 77.8 |

| | | Caldwell | | | | B393 | | | |
|---|---|---|---|---|---|---|---|---|---|
| Treatment Date | Treatment Rate (lb/A) | Sterility (%) | Grain Yield (bu/A) | Hybrid Seed (%) | Seed Germination (%) | Sterility (%) | Grain Yield (bu/A) | Hybrid Seed (%) | Seed Germination (%) |
| 5) D7 | 2.50 | 17 | 56.7 | 7.0 | 87.4 | 64 | 37.7 | 19.7 | 88.4 |
| D7 | 5.00 | 32 | 40.7 | 20.5 | 82.7 | 48 | 35.2 | 22.0 | 90.2 |
| D7 | 7.50 | 32 | 35.8 | 15.7 | 85.3 | 49 | 37.00 | 24.3 | 85.8 |
| D7 | 10.00 | 30 | 32.8 | 16.7 | 75.6 | 40 | 33.8 | 20.0 | 81.8 |
| D7 | 12.50 | 19 | 38.9 | 13.0 | 74.5 | 37 | 32.5 | 19.3 | 93.3 |

## Example 38

The compound of Example 6 was tested in accordance with the procedure of Example 37.

| | | Caldwell | | | | B393 | | | |
|---|---|---|---|---|---|---|---|---|---|
| Treatment Date | Treatment Rate (lb/A) | Sterility (%) | Grain Yield (bu/A) | Hybrid Seed (%) | Seed Germination (%) | Sterility (%) | Grain Yield (bu/A) | Hybrid Seed (%) | Seed Germination (%) |
| Control | | 0 | 78.5 | 0.0 | 81.3 | 0 | 75.3 | 0.1 | 88.0 |
| D1 | 2.50 | 24 | 56.6 | 2.3 | 80.0 | 53 | 67.2 | 12.0 | 88.9 |
| D1 | 5.00 | 71 | 41.3 | 12.7 | 83.6 | 58 | 46.3 | 17.0 | 83.6 |
| D1 | 7.50 | 69 | 33.4 | 14.3 | 87.1 | 85 | 42.9 | 27.3 | 80.4 |
| D1 | 10.00 | 69 | 30.5 | 18.7 | 78.2 | 78 | 44.8 | 17.7 | 84.0 |
| D1 | 12.50 | 81 | 27.2 | 19.7 | 85.8 | 96 | 32.9 | 34.3 | 80.9 |

| | | Caldwell | | | | B393 | | | |
|---|---|---|---|---|---|---|---|---|---|
| Treatment Date | Treatment Rate (lb/A) | Sterility (%) | Grain Yield (bu/A) | Hybrid Seed (%) | Seed Germination (%) | Sterility (%) | Grain Yield (bu/A) | Hybrid Seed (%) | Seed Germination (%) |
| D3 | 2.50 | 20 | 60.8 | 2.7 | 85.3 | 27 | 68.0 | 3.0 | 86.0 |
| D3 | 5.00 | 56 | 41.1 | 20.0 | 78.7 | 94 | 53.2 | 15.3 | 85.8 |
| D3 | 7.50 | 83 | 25.4 | 27.7 | 71.1 | 89 | 42.2 | 24.7 | 83.6 |
| D3 | 10.00 | 99 | 15.7 | 24.3 | 69.3 | 99 | 37.3 | 47.7 | 73.8 |
| D3 | 12.50 | 89 | 21.3 | 26.3 | 74.2 | 98 | 30.3 | 45.0 | 82.2 |
| D5 | 2.50 | 9 | 66.9 | 4.5 | 68.0 | 46 | 58.0 | 17.0 | 90.2 |
| D5 | 5.00 | 39 | 46.4 | 10.3 | 80.0 | 93 | 38.9 | 33.3 | 85.3 |
| D5 | 7.50 | 69 | 30.3 | 16.7 | 76.4 | 93 | 33.0 | 48.7 | 81.8 |
| D5 | 10.00 | 73 | 21.4 | 41.7 | 64.9 | 97 | 29.6 | 38.3 | 78.2 |
| D5 | 12.50 | 86 | 16.9 | 38.0 | 78.2 | 99 | 24.1 | 56.7 | 80.4 |
| D7 | 2.50 | 13 | 58.9 | 15.0 | 80.0 | 63 | 43.4 | 43.7 | 79.6 |
| D7 | 5.00 | 45 | 32.8 | 18.0 | 76.4 | 79 | 28.8 | 47.0 | 77.3 |
| D7 | 7.50 | 86 | 16.3 | 33.7 | 78.7 | 82 | 23.9 | 30.0 | 78.7 |
| D7 | 10.00 | 68 | 17.4 | 34.00 | 74.7 | 83 | 20.2 | 40.7 | 77.8 |
| D7 | 12.50 | 67 | 20.2 | 16.0 | 77.3 | 81 | 18.3 | 37.3 | 84.0 |

## Example 39

The compound of Example 21 was tested in accordance with the procedure of Example 37.

| Treatment Date | Treatment Rate (lb/A) | Caldwell Sterility (%) | Caldwell Grain Yield (bu/A) | Caldwell Hybrid Seed (%) | Caldwell Seed Germination (%) | B393 Sterility (%) | B393 Grain Yield (bu/A) | B393 Hybrid Seed (%) | B393 Seed Germination (%) |
|---|---|---|---|---|---|---|---|---|---|
| Control | | 0 | 78.5 | 0.0 | 81.3 | 0 | 75.3 | 0.1 | 88.0 |
| D1 | 2.50 | 30 | 53.7 | 6.0 | 82.7 | 37 | 58.8 | 9.0 | 87.1 |
| D1 | 5.00 | 42 | 36.2 | 3.7 | 78.7 | 53 | 52.7 | 5.0 | 89.3 |
| D1 | 7.50 | 56 | 40.3 | 4.7 | 81.3 | 79 | 49.0 | 6.3 | 62.7 |
| D1 | 10.00 | 60 | 35.8 | 10.0 | 78.7 | 81 | 41.5 | 26.3 | 89.3 |
| D1 | 12.50 | 86 | 26.6 | 14.3 | 72.0 | 92 | 43.8 | 22.7 | 87.1 |
| D3 | 2.50 | 46 | 50.9 | 10.7 | 80.0 | 44 | 62.5 | 2.3 | 93.3 |
| D3 | 5.00 | 62 | 33.7 | 16.3 | 79.6 | 65 | 48.1 | 10.7 | 90.2 |
| D3 | 7.50 | 52 | 25.6 | 21.3 | 77.3 | 79 | 43.0 | 23.0 | 89.3 |
| D3 | 10.00 | 84 | 22.3 | 16.0 | 79.6 | 85 | 39.2 | 15.0 | 86.2 |
| D3 | 12.50 | 85 | 17.8 | 22.3 | 65.3 | 86 | 35.9 | 30.7 | 87.1 |

| Treatment Date | Treatment Rate (lb/A) | Caldwell Sterility (%) | Caldwell Grain Yield (bu/A) | Caldwell Hybrid Seed (%) | Caldwell Seed Germination (%) | B393 Sterility (%) | B393 Grain Yield (bu/A) | B393 Hybrid Seed (%) | B393 Seed Germination (%) |
|---|---|---|---|---|---|---|---|---|---|
| D5 | 2.50 | 40 | 42.9 | 10.7 | 79.1 | 66 | 51.5 | 16.7 | 88.9 |
| D5 | 5.00 | 63 | 38.1 | 18.7 | 70.1 | 82 | 42.1 | 31.7 | 83.6 |
| D5 | 7.50 | 53 | 25.8 | 22.7 | 71.1 | 88 | 29.1 | 43.0 | 78.7 |
| D5 | 10.00 | 74 | 21.5 | 23.3 | 68.9 | 88 | 30.6 | 51.0 | 79.1 |
| D5 | 12.50 | 76 | 24.0 | 27.7 | 75.1 | 75 | 35.5 | 26.3 | 83.1 |
| D7 | 2.50 | 18 | 49.8 | 16.5 | 84.0 | 43 | 47.6 | 23.3 | 86.2 |
| D7 | 5.00 | 24 | 34.8 | 28.5 | 86.0 | 69 | 28.0 | 29.0 | 81.3 |
| D7 | 7.50 | 43 | 26.1 | 11.3 | 84.9 | 57 | 34.2 | 19.7 | 84.4 |
| D7 | 10.00 | 31 | 27.5 | 18.7 | 68.4 | 70 | 23.0 | 44.3 | 83.6 |
| D7 | 12.50 | 62 | 22.6 | 21.3 | 80.4 | 69 | 23.3 | 51.7 | 84.0 |

Although this invention has been described with respect to specific embodiments, the details thereof are not to be construed as limitations, for it will be apparent that various embodiments, changes and modifications

may be resorted to without departing from the spirit and scope thereof and it is understood that such equivalent embodiments are intended to be included within the scope of this invention.

## Claims

1. A process for selectively sterilizing the male gametes of a plant which comprises applying to a plant locus, a compound selected from the group consisting of:

(a) 2,6-bis(trifluoromethyl)-3-methoxycarbonyl-4-hydroxypyridine;

(b) an agronomically acceptable salt of 2,6-bis(trifluoromethyl)-3-methoxycarbonyl-4-hydroxypyridine; and

(c) 2,6-bis(trifluoromethyl)-3-methoxycarbonyl-4-(organic ester radical)pyridine which is hydrolyzable to 2,6-bis(trifluoromethyl)-3-methoxycarbonyl-4-hydroxypyridine.

2. The process of Claim 1 wherein said salt is the diisopropylamine salt.

3. The process of Claim 1 wherein said salt is the sodium salt.

4. The process of Claim 1 wherein said organic ester radical is a carbonyloxy ester radical.

5. The process of Claim 4 wherein the organic ester is 2,6-bis(trifluoromethyl)-3-(methoxycarbonyl)-4-acetyloxy pyridine.

6. The process of Claim 4 wherein the organic ester is 2,6-bis(trifluoromethyl)-3-(methoxycarbonyl)-4-(4′-trifluoromethylbenzoyloxy)pyridine.

7. The process of Claim 4 wherein the organic ester is 2,6-bis(trifluoromethyl)-3-(methoxycarbonyl)-4-(phenoxycarbonyloxy)pyridine.

8. The process of Claim 1 wherein the organic ester is 2,6-bis(trifluoromethyl)-3-(methoxycarbonyl)-4-(4′-methylphenylsulfonyloxy)pyridine.

9. The process of Claim 1 wherein the organic ester is 2,6-bis(trifluoromethyl)-3-(methoxycarbonyl)-4-(diethoxyphosphinyloxy)pyridine.

10. The process of Claim 1 wherein the compound is applied to the plant locus at a time after initiation of sexual development but before sexual maturity.

11. The process of Claim 1 wherein the plant is a monocotyledon.

12. The process of Claim 1 wherein the plant is wheat.

13. The process of Claim 1 wherein the compound is applied to the plant locus at a rate of about 1 to about 20 lb/a.

14. A process for selectively sterilizing the male gametes of a plant which comprises applying to a plant locus a compound selected from the group consisting of 2,6-bis(trifluoromethyl)-3-(methoxycarbonyl)-4-hydroxypyridine and an agronomically acceptable salt thereof.

15. The process of Claim 14 wherein said salt is the diisopropylamine salt.

16. A process for selectively sterilizing the male gametes of a plant which comprises applying to a plant locus 2,6-bis(trifluoromethyl)-3-(methoxycarbonyl)-4-hydroxypyrdine.

17. A process for production of hybrid seeds which comprises

(i) applying to a plant locus, a compound selected from the group consisting of:

(a) 2,6-bis(trifluoromethyl)-3-methoxy carbonyl-4-hydroxypyridine;

(b) an agronomically acceptable salt of 2,6-bis(trifluoromethyl)-3-methoxycarbonyl-4-hydroxypyridine; and

(c) 2,6-bis(trifluoromethyl)-3-methoxycarbonyl-4-(organic ester radical) pyridine which is hydrolyzable to 2,6-bis(trifluoromethyl)-3-methoxycarbonyl-4-hydroxypyridine.

(ii) causing the female gametes of the plant to be fertilized with pollen from a different cultivar of the same plant species and

(iii) subsequently harvesting the hybrid seeds from said plant.